# EUROPEAN PATENT APPLICATION

(11) **EP 2 759 312 A1**
(43) Date of publication of application: **30.07.2014**
(21) Application number: 12834360.5
(22) Date of filing: 20.09.2012
(51) Int. Cl.: A61M 5/00

(54) **MEDICAL APPARATUS ADMINISTRATION DEVICE AND MEDICAL APPARATUS ADMINISTRATION METHOD**

(30) Priority: 22.09.2011 JP 2011207730
(71) Applicant: Terumo Kabushiki Kaisha, Tokyo 151-0072 (JP)
(72) Inventor: ASAMA, Koichiro, Ashigarakami-gun Kanagawa 259-0151 (JP); TOUNOOKA, Hiroko, Kanagawa 257-0014 (JP)
(74) Representative: Casalonga
(86) International application number: PCT/JP2012/005987
(87) International publication number: WO 2013/042369

(57) **Abstract**

There is provided a medical equipment management apparatus or the like which obtains information for suitably out responding to an alarm from a medical equipment and can perform CQI based on the information related to the timing in which the responding operation is performed.

The medical equipment management apparatus 10 obtains responding time information related to the time until a medical equipment 2a, arranged in a different region, which has output abnormal information is reset to a normal state, together with region information related to a region in which the medical equipment 2a is arranged and responding operation timing information, produces region and responding timing related responding time information 46a for displaying responding time information in relation to the region information and the responding operation timing information of the medical equipment, and display the region and responding timing related responding time information on a display 4a of a personnel side terminal 3a.

## Description

### Technical Field

The present invention relates to a medical equipment management apparatus and a medical equipment management method for managing a medical equipment, for example, an infusion pump.

### Background Art

Conventionally, when liquid medicine is administered to a patient by drip infusion or the like in hospitals, a medical equipment such as an infusion pump is used to correctly control a dose.

The infusion pump includes a transfusion tube and is configured so as a liquid medicine to flow inside the infusion tube. It is configured that when the infusion tube is clogged or is in a similar state, an alert (alarm) is output (e.g., see Patent Literature 1).

Therefore, an assigned medical care worker such as a nurse can carry out an operation, in response to the alert (alarm), such as removing the clogging for resetting.

### Citation List

### Patent Literature

Patent Literature 1: JP 11-137676 A

### Summary of Invention

### Technical Problem

However, there are differences in the number of a medical care worker, the experience which arranged a medical care worker has, or the like among areas such as an ICU (Intensive Care Unit), a General Ward, or the like in which a medical equipment such as an infusion pump is arranged. So that even when the medical equipment such as an infusion pump or the like output an alert, the elapsed time before the medical equipment restarts normally, responding to the alert, is not always constant, which means that the quality of medical care is not uniformly provided.

Further, conventionally, even though it can be vaguely recognized that such ununiformity of quality exists, there is no suitable method to improve the quality of medical care so that improvement is extremely difficult.

Regarding such problem, the object of the present invention is to provide a medical equipment management apparatus and a medical equipment management method which obtain information for suitably responding to an alarm from a medical equipment and can perform CQI (Continuous Quality Improvement) based on the information.

### Solution to Problem

In the present invention, the object mentioned above can be achieved by a medical equipment management apparatus configured to obtain the responding time information related to the time until a medical equipment, each arranged in a different region, which has output abnormal information is reset to a normal state, together with the region information related to a region in which the medical equipment is arranged and the responding operation timing information related to the timing in which the responding operation is performed, produce the region and responding timing related responding time information for displaying the responding time information in relation to the region information and the responding operation timing information of the medical equipment, and display the region and responding timing related responding time information on a display of the personnel side terminal.

According to the configuration, the region and responding timing related responding time information is presented in relation to the region information and the responding operation timing information of the medical equipment.

Therefore, a personnel who visually recognizes the region and responding timing related responding time information such as a doctor or the like can immediately understand how the responding time information differs in relation to the region information, for example, an ICU (Intensive Care Unit) and an OPE (operating room) and the responding timing information, for example, during the morning. So that the personnel can know, for example, suitable number of medical care worker such as a nurse to be arranged in the region such as the ICU (Intensive Care Unit), the OPE (operating room), or the like, thereby allowing to perform the CQI (Continuous Quality Improvement).

Preferably, the responding time information is a time information related to the time until the medical equipment is reset to a normal state with the medicine of which type is the same as the medicine used by the medical equipment when the abnormal information has been output.

According to the configuration, since the responding time information is the time information until the medical equipment is reset to a normal state with the medicine of which type is the same as the medicine used by the medical equipment when the abnormal information has been output, the time information related to a case when the medical equipment is reset to a normal state with the medicine changed after the abnormal information has been output is eliminated.

Therefore, correct responding time information related to the time until being reset to a normal state can be obtained.

Preferably, standard labor cost information of a performer who has performed the responding operation is included, and the responding time labor cost information which is a labor cost of the performer who has performed the responding operation is obtained based on the responding time information and the standard labor cost information, and that the responding time labor cost information is displayed together with the region and responding timing related responding time information.

According to the configuration, since the responding time labor cost information is displayed together with the region and responding timing related responding time information, a personnel such as a doctor who visually recognizes the information can immediately study the suitable number of medical care worker such as a nurse to be arranged in a region such as the ICU (Intensive Care Unit), the OPE (operating room), or the like also from a view point of cost.

Preferably, objective assessment information of the performer who has performed the responding operation is produced based on the responding time information, and the objective assessment information is displayed together with the region and responding timing related responding time information.

According to the configuration, the objective assessment information is displayed together with the region and responding timing related responding time information, a personnel such as a doctor who visually recognizes the information can obtain objective assessment information regarding the suitable number of medical care worker such as a nurse to be arranged in a region such as the ICU (Intensive Care Unit), the OPE (operating room), or the like.

Preferably, the objective assessment information is an assessment information of a responding time length of the performer who has performed the responding operation.

According to the configuration, since the objective assessment information is an assessment information of the responding time length of the performer who has performed the responding operation, a personnel such as a doctor who visually recognizes the information can easily understand that the situation such as a lack of the number of workers exists.

Preferably, the objective assessment information is the information of degree of distribution of a plurality of pieces of responding time information included in the responding time information.

According to the configuration, since the objective assessment information is the information of degree of distribution of the plurality of pieces of responding time information included in the responding time information, it is understood that there is difference in skill for each performer who has performed the responding operation. So the fact can easily be understood that training is not thoroughly done.

Preferably, the objective assessment information is the information of disparity among the pieces of responding time information of the different pieces of responding operation timing information.

According to the configuration, since the objective assessment information is the information of disparity among the pieces of responding time information of the different pieces of responding operation timing information, a mismatch or the like of arrangement of workers in the area for a time zone or the like can easily be understood.

In the present invention, the object mentioned above can be achieved by a medical equipment management method configured to obtain the responding time information related to the time until a medical equipment, each arranged in a different region, which has output abnormal information is reset to a normal state, together with the region information related to a region in which the medical equipment is arranged and the responding operation timing information related to the timing in which the responding operation is performed, produce the region and responding timing related responding time information for displaying the responding time information in relation to the region information and the responding operation timing information of the medical equipment, and display the region and responding timing related responding time information on a display of the personnel side terminal.

### Advantageous Effects of Invention

As described above, according to the present invention, a medical equipment management apparatus and a medical equipment management method which can obtain information for suitably carrying out an operation responding to an alarm from the medical equipment, and can perform the CQI (Continuous Quality Improvement) based on the information.

### Brief Description of Drawings

Fig. 1 is a schematic view illustrating a pump CQI system according to an embodiment including a medical equipment management apparatus of the present invention such as a management server.
Fig. 2 is a schematic block diagram illustrating a main configuration of the infusion pump in Fig. 1.
Fig. 3 is a schematic view illustrating a main configuration of the management server in Fig. 1.
Fig. 4 is a schematic block diagram illustrating a content of each memorizing unit in Fig. 3.
Fig. 5 is a schematic flow chart illustrating a main operation or the like of the CQI system according to the embodiment.
Fig. 6 is a schematic flow chart illustrating a main operation of the CQI system according to the embodiment.
Fig. 7 is a schematic explanatory drawing illustrating an example of "time and area related responding time data" in Fig. 2.
Fig. 8 is a schematic explanatory drawing illustrating an example of "average responding time and standard deviation data for each area and time zone".
Fig. 9 is a schematic explanatory drawing illustrating a chart of "alert responding time analysis data".
Fig. 10 is a schematic explanatory drawing illustrating an exemplary data displayed on a terminal side display of a medical office terminal.

### Description of Embodiments

A preferable embodiment according to the present invention will be described below in detail referring to attached drawings or the like.

Note that, since the embodiment described below is a preferable specific example of the present invention, various technically preferable limitations are applied. However, the scope of the present invention is not limited to these aspects otherwise particularly specified that such limitation limits the present invention in the description below.

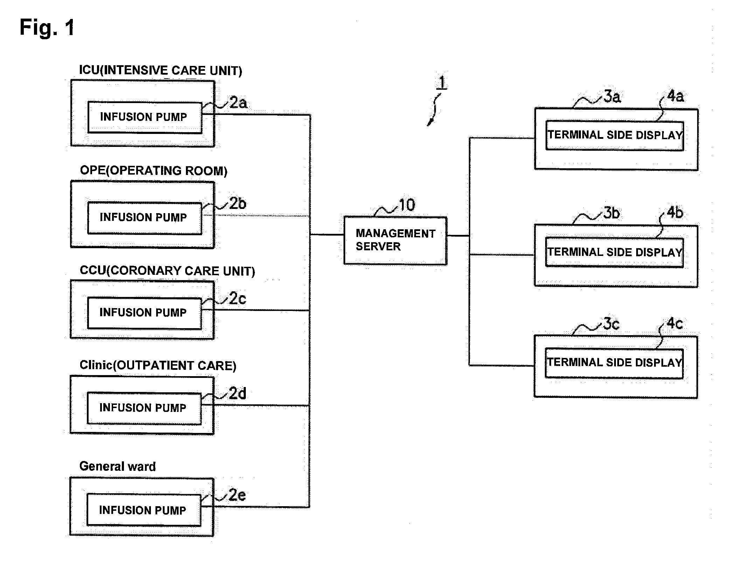

Fig. 1 is a schematic view illustrating a pump CQI (Continuous Quality Improvement) system 1 according to an embodiment including a medical equipment management apparatus of the present invention, for example, a management server 10.

As illustrated in Fig. 1, the pump CQI system 1 includes infusion pumps 2a to 2e or the like arranged in a region (area) such as an ICU (Intensive Care Unit), an OPE (operating room), a CCU (Coronary Care Unit), a Clinic (outpatient care), a General ward, or the like.

The infusion pump 2a or the like is a medical equipment used together with an equipment such as a drip infusion for correctly administering medicines to a patient.

Note that, even though the embodiment is described using the infusion pump 2a or the like, the medical equipment according to the present invention may be a syringe pump or the like, other than the infusion pump 2a or the like.

As illustrated in Fig. 1, the infusion pump 2a or the like is communicatably connected to the management server 10, and also communicatably connected to, for example, medical office terminals 3a to 3c which are personnel side terminals arranged in the medical office or the like for a doctor or the like.

Note that, in the medical office terminal 3a or the like, terminal side displays 4a to 4c, each being a display, are formed.

Note that, in the embodiment, for convenience of description, an example in which the infusion pump 2 or the like is directly connected to the management server 10 as illustrated in Fig. 1 is described. However, the present invention is not limited to such configuration and may have a configuration in which each infusion pump 2a or the like is connected to the management server via a "pump communication software (gateway terminal)" which manages the infusion pump 2a or the like. In this case, the "pump communication software (gateway terminal)" and the "management server" share the function of the management server 10 of the embodiment.

The pump CQI system 1 according to the embodiment is a system for putting forward continuous medical quality improvement related to the infusion pump 2a or the like. When abnormal information, for example, an alert (alarm) from the infusion pump 2a or the like is output from an operation history (pump history) collected from the infusion pump 2a or the like, the pump CQI system 1 analyzes a responding time consumed during a resetting operation for setting the operation to a normal state, or the like, so as to perform improvement in, for example, arrangement of the suitable number of a medical care worker such as a nurse who is to be arranged in the ICU (Intensive Care Unit), the OPE (operating room), or the like in which the infusion pump 2a or the like is set.

The infusion pump 2a or the like, the management server 10, and the medical office terminal 4a or the like illustrated in Fig. 1 include a computer. The computer includes a CPU (Central Processing Unit), a RAM (Random Access Memory), a ROM (Read Only Memory), or the like, not shown in the drawing, which is connected via a bus.

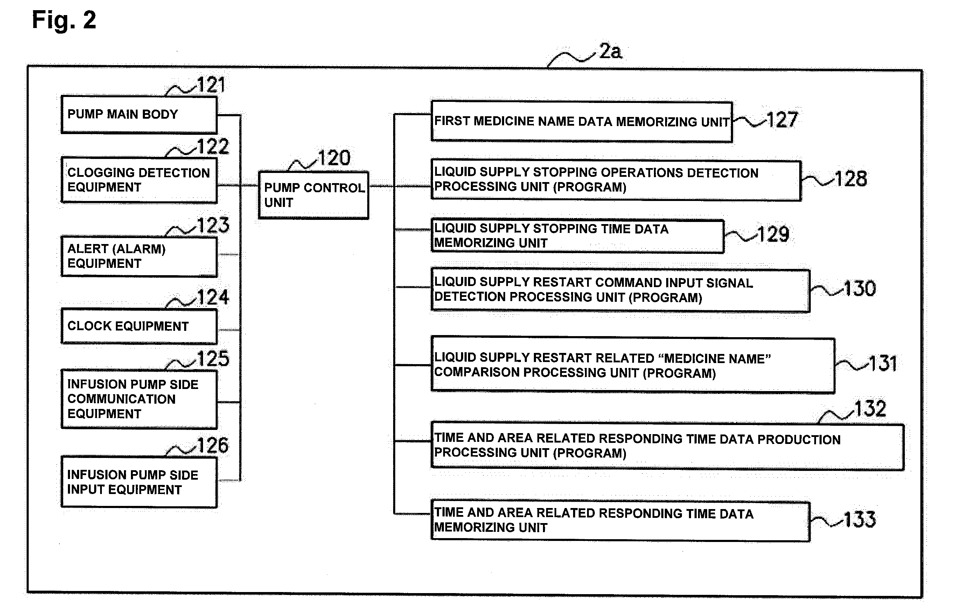

Fig. 2 is a schematic block diagram illustrating a main configuration of the infusion pump 2a or the like in Fig. 1.

As illustrated in Fig. 2, the infusion pump 2a or the like includes a pump control unit 120 and performs an operation of administering liquid medicine by drip infusion or the like to a patient at a predetermined administration rate. The infusion pump 2a or the like also includes a pump main body 121 which performs function of stopping the operation of the infusion pump 2a or the like when an abnormal state such as clogging in a transfusion tube of the infusion pump 2a or the like occurs, and a clogging detection equipment 122 having a sensor or the like which detects clogging or the like when such happens in the infusion pump 2a or the like during administration of liquid medicine.

Further, the infusion pump 2a or the like includes an alert (alarm) equipment 123 which detects an abnormal state when the clogging detection equipment 122 detects a clogging or the like in the infusion pump 2a or the like, a clock equipment 124 which is a clock or the like, a pump side communication equipment 125 which allows the infusion pump 2a or the like to communicate with the management server 10 or the like, and an infusion pump side input equipment 126 for inputting each type of signal or the like to the infusion pump 2a or the like.

These pump main body 121 and the like are connected to the pump control unit 120 as illustrated in Fig. 1 and are controlled.

Note that, the pump control unit 120 also controls each type of memorizing unit and each type of processing unit (program) as illustrated in Fig. 1, and these configurations will be described below.

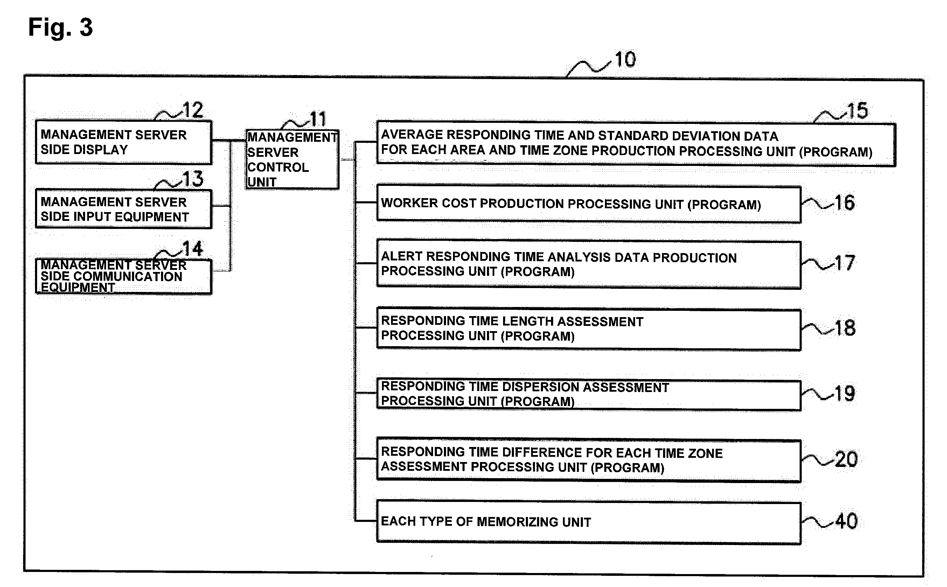

Fig. 3 is a schematic view illustrating a main configuration of the management server 10 in Fig. 1.

As illustrated in Fig. 3, the management server 10 includes a management server control unit 11, a display for displaying each type of data, for example, a management server side display 12, a management server side input equipment 13 for inputting each type of data, and a management server side communication equipment 14 which allows the management server 10 to communicates with the infusion pump 2a or the like or the medical office terminal 3a or the like.

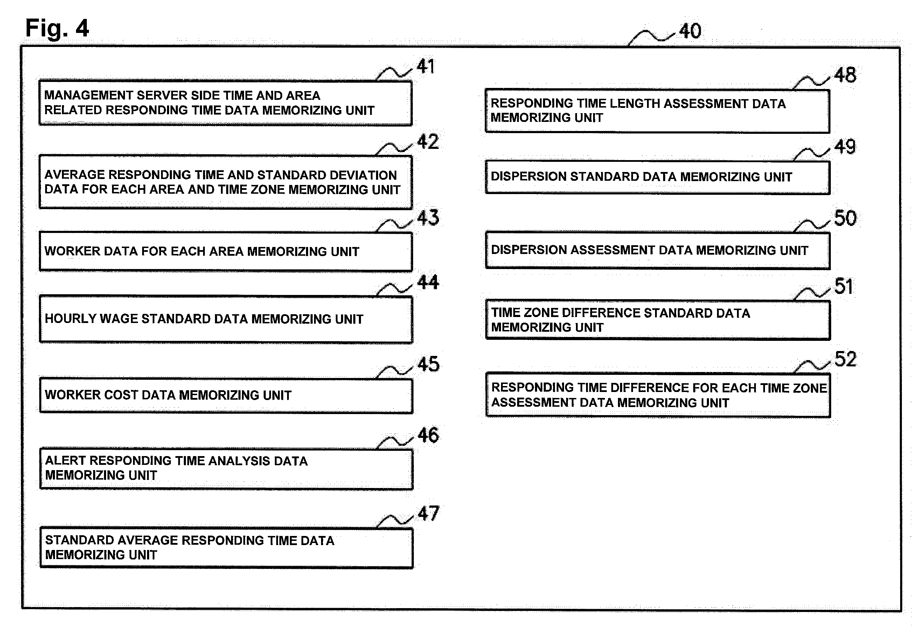

These management server side display 12 and the like are connected to the management server control unit 11 and controlled. Further, as illustrated in Fig. 3, the management server control unit 11 has a configuration which also controls each type of memorizing unit 40 and each type of processing unit (program). The content of each type of memorizing unit 40 and each type of processing unit (program) will be described below. Note that, Fig. 4 is a schematic block diagram illustrating a content of each memorizing unit 40 in Fig. 3 of which content will be described below.

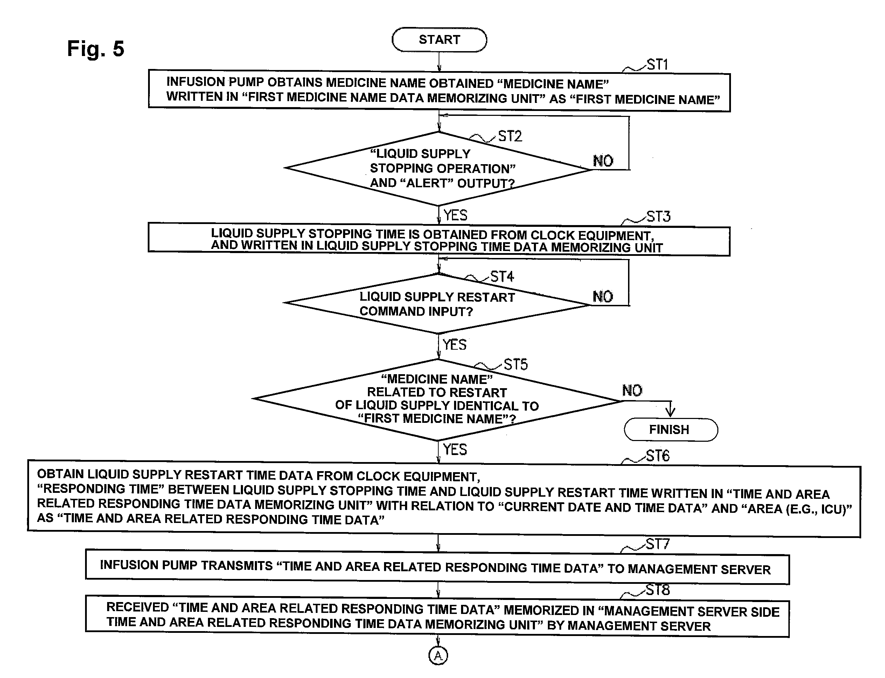

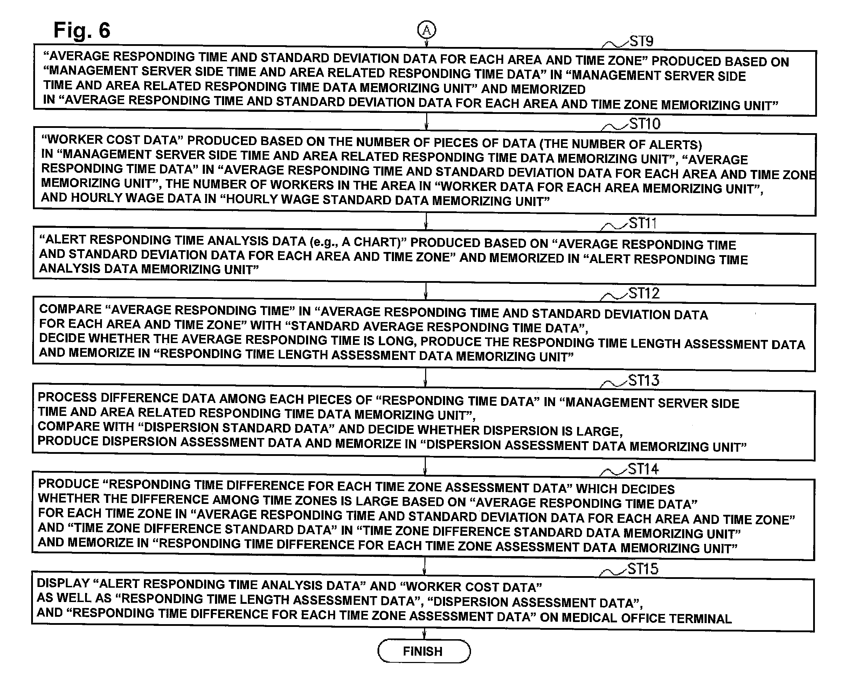

Fig. 5 and Fig. 6 are schematic flow charts illustrating main operations or the like of the CQI system 1 according to the embodiment. The embodiment will be described along with flow charts in Fig. 5 and Fig. 6, and configurations in Figs. 1 to 4 will be described below.

First, in step (hereinafter referred to as "ST") 1 in Fig. 5, the power switch of the infusion pump 2a or the like in Fig. 1 is "ON", and when a medicine, for example, "Diprivan (a trademark of AstraZeneca K.K.)" which is an anesthetic is set in the infusion pump 2a or the like, the infusion pump 2a or the like makes the data of the medicine name "Diprivan" to be memorized in a "first medicine name data memorizing unit 127" in Fig. 2.

Then, the step proceeds to ST2. In ST2, the liquid supply stopping operations detection processing unit (program) 128 in Fig. 2 operates to decide whether a "liquid supply stopping operation" and an "alert" have been output. That is, when a clogging or the like occurs in a transfusion tube or the like of the infusion pump 2a or the like so that the clogging detection equipment 122 detects the clogging and an alert (alarm) equipment 123 outputs an alert, the pump main body 121 simultaneously performs stopping operation of liquid supply of the liquid medicine "Diprivan".

When it is decided in ST2 that the infusion pump 2a or the like has output an alert and simultaneously has carried out a stopping operation of liquid supply of the liquid medicine "Diprivan", the step proceeds to ST3.

In ST3, the infusion pump 2a or the like obtains the liquid supply stopping time from the clock equipment 124 in Fig 2 and makes the liquid supply stopping time data to be memorized in a the liquid supply stopping time data memorizing unit 129.

Then the step proceeds to ST4. In ST4, whether the liquid supply restart command has been input to the infusion pump 4a or the like is decided.

Specifically, for example, when an alert is output from the infusion pump 2a or the like arranged in the ICU (Intensive Care Unit) and, thereby, the liquid supply operation stops, a medical care worker such as a nurse or the like arranged in the ICU checks the infusion pump 2a or the like and carries out processing such as refilling with a new liquid medicine "Diprivan" so as to restart (reset) the liquid supply operation of the infusion pump 2a or the like in a normal state.

Then, in ST4, the liquid supply restart command input signal detection processing unit (program) 130 in Fig. 2 decides whether such liquid supply restart command has been input to the infusion pump 2a or the like.

In ST4, when the liquid supply restart command is input, the step proceeds to ST5. In ST5, it is decided whether the medicine name related to the restart of liquid supply is identical to (one of those of the same type of) the first medicine name.

Specifically, a "liquid supply restart related "medicine name" comparison processing unit (program) 131" in Fig. 2 operates, in the embodiment, for example, to decide whether the medicine name is the same name as "Diprivan".

As described above, the embodiment is configured so that the case when liquid supply is restarted with a different medicine being set is not taken into account, but only the case when liquid supply restarts with the same medicine is taken into account. Thereby, only the case when correct response to the alert is performed is taken into account so that reliability of the data of "responding time" which will be described below is improved.

In ST5, when it is decided that the medicine name related to the restart of liquid supply is identical to the first medicine name, the step proceeds to ST6.

In ST6, a "time and area related responding time data production processing unit (program) 132" in Fig. 2 operates to obtain "liquid supply restart time data" with reference to the clock equipment 124.

Then, the time between the "liquid supply restart time data" and the "liquid supply stopping time data" in the "liquid supply stopping time data memorizing unit 129" in Fig. 2 is made to be memorized in a "time and area related responding time data memorizing unit 133" in Fig. 2 as a "responding time", and the "liquid supply restart time data" is made to be memorized in the "time and area related responding time data memorizing unit 133" in Fig. 2 as "time and area related responding time data 133a" with relation to "current date (e.g., January, 15, 2010) and time data (e.g., 8:00 to 8:30)" and "area (e.g., ICU)".

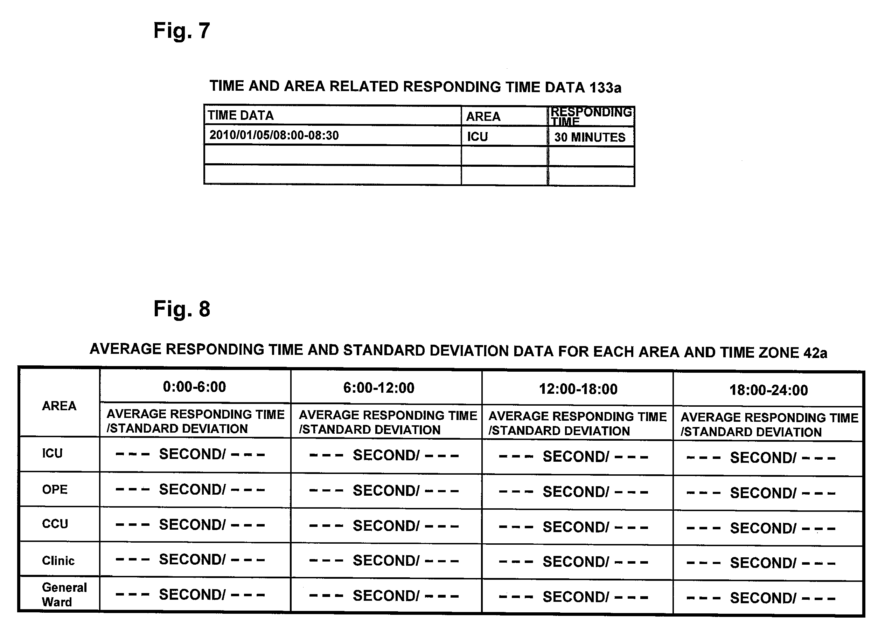

Fig. 7 is a schematic explanatory drawing illustrating an example of "time and area related responding time data 133a" in Fig. 2. As illustrated in Fig. 7, the time data is memorized as "January 15, 2010", the area as "ICU", and the responding time as "30 minutes".

As described above, the "time data" is an example of the responding operation timing information, the "ICU" is an example of the region information, and the "responding time" is an example of the responding time information.

In this manner, the infusion pump 2a or the like obtains data of the responding time of a medical care worker such as a nurse or the like in the area (e.g., ICU) related to the time from the "liquid supply stop" and the "alert output" to the following "liquid supply restart (reset)".

Then, the step proceeds to ST7. In ST7, the infusion pump 2a or the like transmits the "time and area related responding time data 133a" illustrated in Fig. 7 to the management server 10 in Fig. 1 via an infusion pump side communication equipment 125 in Fig. 2.

Then, the step proceeds to ST8. In ST8, the management server 10 makes the "time and area related responding time data 133a" received from the infusion pump 2a or the like to be memorized in a "management server side time and area related responding time data memorizing unit 41" in Fig. 4.

Then, the step proceeds to ST9. In ST9, an "average responding time and standard deviation data for each area and time zone production processing unit (program) 15" in Fig. 3 operates to produce "average responding time and standard deviation data for each area and time zone 42a" based on "management server side time and area related responding time data" in the "management server side time and area related responding time data memorizing unit 41" in Fig. 4 and to make the "average responding time and standard deviation data for each area and time zone 42a" to be memorized in an "average responding time and standard deviation data for each area and time zone memorizing unit 42".

Fig. 8 is a schematic explanatory drawing illustrating an example of "average responding time and standard deviation data for each area and time zone 42a".

As illustrated in Fig. 8, the data 42a includes the average responding time and standard deviation for each area (e.g., ICU) and time zone (e.g., 0:00 to 6:00).

That is, the "average responding time and standard deviation data for each area and time zone 42a" is produced by processing based on the "time and area related responding time data 133a" in Fig. 7.

Then, the step proceeds to ST10. In ST10, a "worker cost production processing unit (program) 16" in Fig. 3 operates to produce "worker cost data" for each area based on the number of pieces of data (that is, the number of alerts) of "time and area related responding time data 133a" in Fig. 7 in the "management server side time and area related responding time data" in Fig. 4, "average responding time data" in "average responding time and standard deviation data for each area and time zone 42a" in Fig. 8, the number of workers in the area in a "worker data for each area memorizing unit 43 (e.g., data of workers arranged in an area such as ICU, or the like)" in Fig. 4, and hourly wage standard data of a medical care worker such as a nurse in an "hourly wage standard data memorizing unit 44" in Fig. 4, and to make the "worker cost data" for each area to be memorized in a "worker cost data memorizing unit 45" in Fig. 4.

That is, the data shows how much labor cost is required, by the "responding time" to reset the infusion pump 2a or the like of a medical care worker such as a nurse or the like, from the data of a worker, an average responding time, an hourly wage, or the like for each area such as an ICU. Therefore, a doctor or the like who visually recognizes the data can rapidly and suitably make assessment whether the arrangement of a worker for each area or the like is appropriate from a view point of cost.

Note that, the "worker cost data" is an example of the responding time labor cost information and the "hourly wage standard data" is an example of the standard labor cost information.

Then, the step proceeds to ST11. In ST11, an "alert responding time analysis data production processing unit (program) 17" in Fig. 3 operates to produce "alert responding time analysis data 46a" based on the "average responding time and standard deviation data for each area and time zone 42a" in Fig 8, and to make the "alert responding time analysis data 46a" to be memorized in an "alert responding time analysis data memorizing unit 46" in Fig. 4.

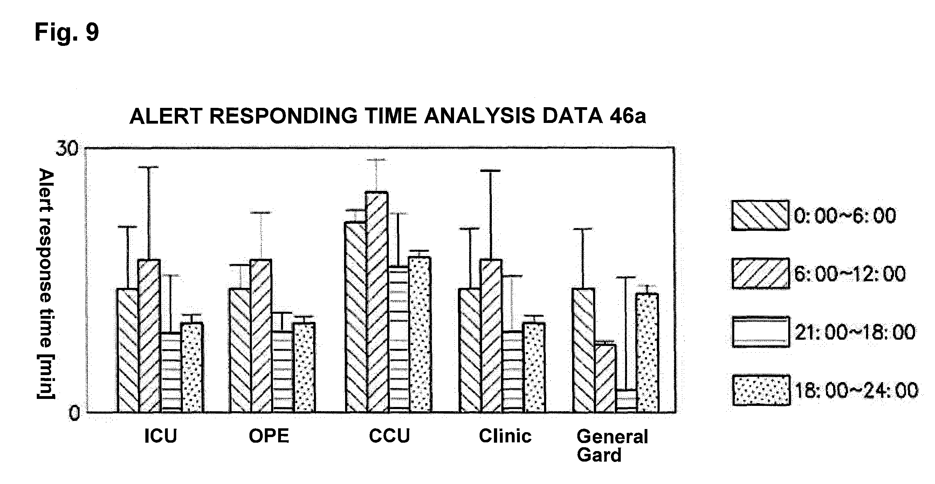

Fig. 9 is a schematic explanatory drawing illustrating a chart of "alert responding time analysis data 46a"

As illustrated in Fig. 9, the "alert responding time analysis data 46a" (chart) shows an average responding time and standard deviation in each area (an ICU (Intensive Care Unit), an OPE (operating room), a CCU (Coronary Care Unit), a Clinic (outpatient care), and a General Ward) for each of the four time zones (0:00 to 6:00, 6:00 to 12:00, 12:00 to 18:00, and 18:00 to 24:00).

Note that, the "alert responding time analysis data 46a" is an example of the region and responding timing related responding time information.

Therefore, a doctor or the like who visually recognizes the "alert responding time analysis data 46a" can understand the situation of the "responding time" for each time zone and each area so that the "alert responding time analysis data 46a" can be used as a material for studying optimum arrangement of a worker for each time zone and each area. Thereby, the CQI (Continuous Quality Improvement) can be performed.

Then, the step proceeds to ST12. In ST12, a responding time length assessment processing unit (program) 18 operates to compare the "average responding time" in the "average responding time and standard deviation data for each area and time zone 42a" in Fig. 8 and the "standard average responding time data" which is the average responding time data, memorized in a "standard average responding time data memorizing unit 47" in Fig. 4, used as an objective standard, decide whether the average responding time is longer or shorter than the standard, produce the responding time length assessment data, and make the responding time length assessment data to be memorized in a "responding time length assessment data memorizing unit 48" in Fig. 4.

That is, by comparing the "average responding time data" for each area and time zone with the "standard average responding time data", decision can be made whether the average responding time data is longer or shorter than the standard value. A doctor or the like who recognizes that the "average responding time data" is longer than the standard by visually recognizing the "responding time length assessment data" can immediately understand the lack of workers in the area or time zone.

Note that, the "responding time length assessment data" is an example of the assessment information of the responding time length (objective assessment information).

Then, the step proceeds to ST13. In ST13, a responding time dispersion assessment processing unit (program) 19 operates to process difference data among each pieces of "responding time data" in the management server side time and area related responding time data memorizing unit 41 in Fig. 4, compare with the "dispersion standard data" in a "dispersion standard data memorizing unit 49" in Fig. 4, decide whether the dispersion is large, produce dispersion assessment data of the dispersion, and make the dispersion assessment data to be memorized in a "dispersion assessment data memorizing unit 50" in Fig. 4.

That is, by visually recognizing the "dispersion assessment data" of the "responding time", a doctor or the like can immediately understand the fact such that there is great difference among medical care workers such as a nurse for each time zone and each area, and also understand that training of the medical care worker is not thoroughly done.

Note that, the "dispersion assessment data" is an example of the information of degree of distribution of a plurality of pieces of responding time information.

Then, the step proceeds to ST14. In ST14, a "responding time difference for each time zone assessment processing unit (program) 20" in Fig. 3 operates to compare the difference data in the "average responding time data" for each time zone in the "average responding time and standard deviation data for each area and time zone 42a" in Fig. 8 and "time zone difference standard data" in a "time zone difference standard data memorizing unit 51" in Fig. 4, decide whether the difference data is larger than the "time zone difference standard data", that is, whether the difference among time zones is large, produce "responding time difference for each time zone assessment data", and make the "responding time difference for each time zone assessment data" to be memorized in a "responding time difference for each time zone assessment data memorizing unit 52" in Fig. 4.

That is, when the difference data of the "average responding time data" for each time zone for each area is large, a doctor or the like who visually recognizes such data can immediately understand that there is a mismatch in arrangement of a worker for each time zone, and can make an improvement plan.

Note that, the "responding time difference for each time zone assessment data" is an example of the information of disparity among the pieces of the responding time information.

Then, the step proceeds to ST15. In ST15, the management server 10 displays the "alert responding time analysis data 46a" in Fig. 9, the "worker cost data" in Fig. 4, or the like on the terminal side display 4a or the like of the medical office terminal 3a or the like in Fig. 1.

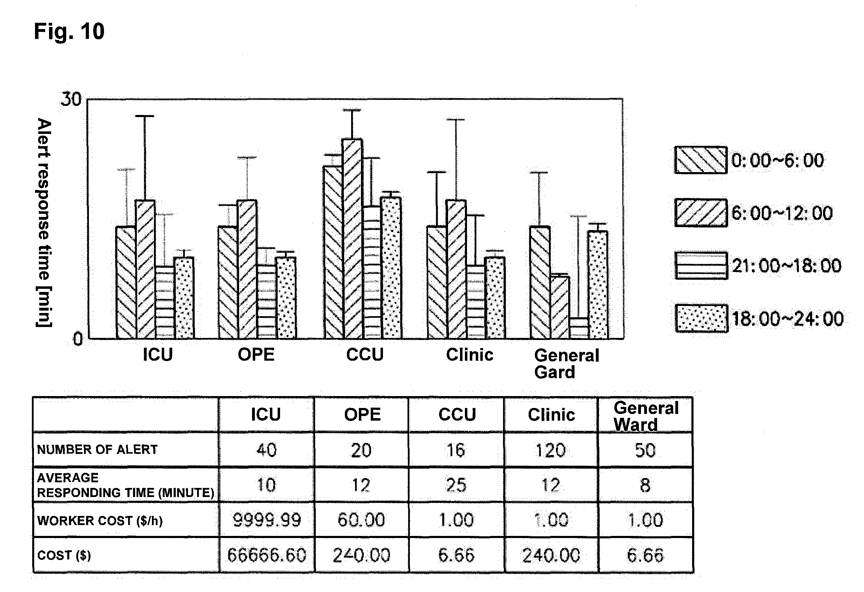

Fig. 10 is a schematic explanatory drawing illustrating an exemplary data displayed on the terminal side display 4a or the like of the medical office terminal 3a or the like.

As illustrated in Fig. 10, the "alert responding time analysis data 46a" and the "worker cost data" are displayed on the terminal side display 4a or the like.

Although not illustrated in Fig. 10, the "responding time length assessment data", the "dispersion assessment data", and the "responding time difference for each time zone assessment data" in Fig. 4 can simultaneously be displayed.

In this case, a doctor or the like who sees the terminal side display 4a or the like can immediately understand, for example, what is to be improved for arrangement of a worker for each area, each time zone, or the like based on the "responding time" to the alert, as described above. Therefore, CQI (Continuous Quality Improvement) can be performed.

The present invention is not limited to each embodiment described above.

### Reference Signs List

- 1: pump CQI system
- 2a to 2e: infusion pump
- 3a to 3c: medical office terminal
- 4a to 4c: terminal side display
- 10: management server
- 11: management server control unit
- 12: management server side display
- 13: management server side input equipment
- 14: management server side communication equipment
- 15: average responding time and standard deviation data for each area and time zone production processing unit (program)
- 16: worker cost production processing unit (program)
- 17: alert responding time analysis data production processing unit (program)
- 18: responding time length assessment processing unit (program)
- 19: responding time dispersion assessment processing unit (program)
- 20: responding time difference for each time zone assessment processing unit (program)
- 40: each type of memorizing unit
- 41: management server side time and area related responding time data memorizing unit
- 42: average responding time and standard deviation data for each area and time zone memorizing unit
- 42a: average responding time and standard deviation data for each area and time zone
- 43: worker data for each area memorizing unit
- 44: hourly wage standard data memorizing unit
- 45: worker cost data memorizing unit
- 46: alert responding time analysis data memorizing unit
- 46a: alert responding time analysis data
- 47: standard average responding time data memorizing unit
- 48: responding time length assessment data memorizing unit
- 49: dispersion standard data memorizing unit
- 50: dispersion assessment data memorizing unit
- 51: time zone difference standard data memorizing unit
- 52: responding time difference for each time zone assessment data memorizing unit
- 120: pump control unit
- 121: pump main body
- 122: clogging detection equipment
- 123: alert (alarm) equipment
- 124: clock equipment
- 125: infusion pump side communication equipment
- 126: infusion pump side input equipment
- 127: first medicine name data memorizing unit
- 128: liquid supply stopping operations detection processing unit
- 129: liquid supply stopping time data memorizing unit
- 130: liquid supply restart command input signal detection processing unit (program)
- 131: liquid supply restart related "medicine name" comparison processing unit (program)
- 132: time and area related responding time data production processing unit (program)
- 133: time and area related responding time data memorizing unit
- 133a: time and area related responding time data

## Claims

1. A medical equipment management apparatus configured to obtain responding time information related to a time until a medical equipment, arranged in a different region, which has output abnormal information is reset to a normal state, together with region information related to a region in which the medical equipment is arranged and responding operation timing information related to a timing in which the responding operation is performed,
produce region and responding timing related responding time information for displaying the responding time information in relation to the region information and the responding operation timing information of the medical equipment, and
display the region and responding timing related responding time information on a display of a personnel side terminal.

2. The medical equipment management apparatus according to claim 1, wherein the responding time information is time information related to the time until the medical equipment is reset to a normal state with a medicine of which type is same as a medicine used by the medical equipment when the abnormal information has been output.

3. The medical equipment management apparatus according to claim 1 further comprises standard labor cost information of a performer who has performed the responding operation, wherein
responding time labor cost information which is a labor cost of a performer who has performed the responding operation based on the responding time information and the standard labor cost information is obtained, and the responding time labor cost information is displayed together with the region and responding timing related responding time information.

4. The medical equipment management apparatus according to claim 2 further comprises standard labor cost information of a performer who has performed the responding operation, wherein
responding time labor cost information which is a labor cost of a performer who has performed the responding operation based on the responding time information and the standard labor cost information is obtained, and the responding time labor cost information is displayed together with the region and responding timing related responding time information.

5. The medical equipment management apparatus according to claim 1, wherein objective assessment information of a performer who has performed the responding operation is produced based on the responding time information, and the objective assessment information is displayed together with the region and responding timing related responding time information.

6. The medical equipment management apparatus according to claim 2, wherein objective assessment information of the performer who has performed the responding operation is produced based on the responding time information, and the objective assessment information is displayed together with the region and responding timing related responding time information.

7. The medical equipment management apparatus according to claim 3, wherein objective assessment information of the performer who has performed the responding operation is produced based on the responding time information, and the objective assessment information is displayed together with the region and responding timing related responding time information.

8. The medical equipment management apparatus according to claim 4, wherein objective assessment information of the performer who has performed the responding operation is produced based on the responding time information, and the objective assessment information is displayed together with the region and responding timing related responding time information.

9. The medical equipment management apparatus according to any one of claim 5 to 8, wherein the objective assessment information is assessment information of a responding time length of the performer who has performed the responding operation.

10. The medical equipment management apparatus according to any one of claim 5 to 8, wherein the objective assessment information is information of degree of distribution of a plurality of pieces of responding time information included in the responding time information.

11. The medical equipment management apparatus according to any one of claim 5 to 8, wherein the objective assessment information is information of disparity among the pieces of responding time information of the different pieces of responding operation timing information.

12. A medical equipment management method configured to obtain responding time information related to a time until a medical equipment, arranged in a different region, which has output abnormal information is reset to a normal state, together with region information related to a region in which the medical equipment is arranged and responding operation timing information related to the timing in which the responding operation is performed,
produce region and responding timing related responding time information for displaying the responding time information in relation to the region information and the responding operation timing information of the medical equipment, and
display the region and responding timing related responding time information on a display of a personnel side terminal.
